# EUROPEAN PATENT APPLICATION

(11) **EP 2 789 332 A1**
(43) Date of publication of application: **15.10.2014**
(21) Application number: 12854772.6
(22) Date of filing: 07.12.2012
(51) Int. Cl.: A61K 31/136, A61K 31/18, A61P 19/10, A61P 19/00

(54) **PHARMACEUTICAL COMPOSITION COMPRISING DIAMINODIPHENYLSULFONE OR THE PHARMACEUTICALLY ACCEPTABLE SALTS THEREOF FOR PREVENTING OR TREATING DISEASES ASSOCIATED WITH DECREASE IN BONE MASS**

(30) Priority: 07.12.2011 KR 20110130608
(71) Applicant: Gachon University of Industry-Academic Cooperation Foundation, Seongnam-si, Gyeonggi-do 461-701 (KR)
(72) Inventor: PARK, Sang Chul, Seongnam-si Gyeonggi-do 463-470 (KR); CHO, Sung Chun, Incheon 403-010 (KR); SON, Young Hoon, Seoul 122-853 (KR); LEE, Seok Jin, Seoul 110-809 (KR); CHUNG, Sun Gun, Seoul 136-020 (KR)
(74) Representative: Ström & Gulliksson AB
(86) International application number: PCT/KR2012/010648
(87) International publication number: WO 2013/085352

(57) **Abstract**

The present invention relates to a pharmaceutical composition for preventing or treating a disease with bone mass loss, which comprises diaminodiphenylsulfone (Dapsone; DDS) or a pharmaceutically acceptable salt thereof as an active ingredient. The composition according to the present invention may be effectively used to prevent or treat a disease with bone mass loss such as osteopenia or osteoporosis by effectively restoring the reduction of bone density and bone mass.

## Description

### [Technical Field]

The present invention relates to diaminodiphenylsulfone (Dapsone; DDS) which can be effectively used for the prevention or treatment of a disease with bone mass loss.

### [Background Art]

Bone is a very dynamic tissue that is decomposed little by little daily and is newly formed in proportion to the decomposed amount to maintain homeostasis. The bond remodeling process that includes osteoclast-mediated bone resportion, osteoblast-mediated bone formation and the resting phase repeatedly occurs over a period of about 120-150 days. For healthy adults, bone resorption and bone formation are tightly controlled, and the total amount of bone mass rarely changes.

However, in diseases with bone mass loss, the balance between bone formation and bone resorption is disrupted, resulting in a decrease in bone mass and deterioration in bone tissue. Further, the decrease in bone mass reaches 20-30% and increases the risk of bone fracture, and it may cause the person to lie on a sickbed, deform the body shape, or lead to death in some cases depending on the fracture site, such as fracture at hip joints.

Typical examples of diseases with bone mass loss include osteoporosis. Osteoporosis is a systemic disease characterized by a decrease in bone mass and deterioration in bone tissue. Osteoporosis is a degenerative bone disease characterized by reduction in bone volume and structural deterioration of bone and leads to the increased risk of bone fracture. It is known that osteoporosis results in the weakening of bone, which causes various bone fractures, particularly femur fracture, which limit long-term activity, makes it impossible to enjoy healthy life, and is responsible for 15% of the deaths of aged people.

It is known that bone mass is influenced by various factors, including genetic factors, nutrition intake, hormonal changes, exercise, and a difference in lifestyle and that osteoporosis is caused by aging, menopause, ovary excision, insufficient exercise, low bodyweight, smoking, low-calcium diet, etc. Because osteoporosis is caused mainly by aging or postmenopausal hormonal imbalance among various causes possibly considered, osteoporosis has been treated mainly by administering estrogen, vitamin D or calcitonin. However, the administration of hormones has a risk of causing cancer (particularly, breast cancer, uterine, etc.), and thus does not appear to be a safe therapeutic method. In addition, bisphosphonate has been used as a second-generation therapeutic agent. However, bisphosphonate has a problem in that rebound occurs when the administration thereof is discontinued. The above-mentioned methods can delay the progression of osteoporosis, but it is difficult to regenerate lost bone by these methods.

Meanwhile, it was reported that diaminodiphenylsulfone (Dapsone; DDS) well-known as an antibiotic increases the life span of *Caenorhabditis elegans* by 20-30%. DDS is a substance synthesized one century ago, is well known as a therapeutic agent for leprosy and is used as an important drug for many other skin diseases.

### [Disclosure]

### [Technical Problem]

The effect of DDS against diseases with bone mass loss has not yet been reported in the literature. Accordingly, the present inventors have found that DDS according to the present invention effectively restores the reduction of bone density and bone mass, and thus can be effectively used for the prevention or treatment of diseases with bone mass loss such as osteopenia or osteoporosis, thereby completing the present invention.

### [Technical Solution]

An object of the present invention is to provide diaminodiphenylsulfone (Dapsone; DDS) or a pharmaceutically acceptable salt thereof for preventing or treating a disease with bone mass loss such as osteopenia or osteoporosis by effectively restoring the reduction of bone density and bone mass.

### [Advantageous Effects]

Diaminodiphenylsulfone (Dapsone; DDS) according to the present invention can be effectively used to prevent or treat a disease with bone mass loss such as osteopenia or osteoporosis by effectively restoring the reduction of bone density and bone mass.

### [Description of Drawings]

FIG. 1 shows the results of numerical comparison indicating the effect of DDS on the restoration of bone density and bone mass in ovariectomy-induced osteoporosis according to an example of the present invention.
FIG. 2 is a set of micro-CT images showing the effect of DDS on the restoration of bone density in ovariectomy-induced osteoporosis according to an example of the present invention.
FIG. 3 shows the effect of DDS on the control of Trabecular thickness and Trabecular separation in ovariectomy-induced osteoporosis according to an example of the present invention.
FIG. 4 shows the results of numerical comparison indicating the effect of DDS on the restoration of bone density and bone mass in osteoporosis caused by aging, according to an example of the present invention.

### [Best Mode]

The present invention provides a pharmaceutical composition for preventing or treating a disease with bone mass loss, comprising diaminodiphenylsulfone or a pharmaceutically acceptable salt thereof.

As used herein, the term "diaminodiphenylsulfone" refers to a compound, which is represented by the following formula 1 and has a general name of Dapsone or a nickname of DDS. The diaminodiphenylsulfone is a white, odorless and crystalline powder insoluble in water. Generally, diaminodiphenylsulfone is administered at a dose of 100 mg/day for the treatment of leprosy. In addition, it is also used for the treatment of dermatitis, rheumatoid arthritis or malaria.

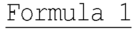

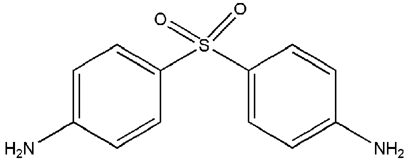

The DDS that is used in the present invention may be commercially available, or may be synthesized or extracted from natural materials, but is not limited thereto. Preferably, the DDS that is used in the present invention may be the one autoclaved after mixing with water in order to further improve the water-insoluble property thereof. The autoclaving may be performed according to a general method known in the art. Preferably, the autoclaving may be performed at a temperature of 121 °C and a pressure of 151 psi for 15-20 minutes.

As used herein, the term "pharmaceutically acceptable salt" refers to a formulation of a compound that does not cause significant irritation to an organism to which it is administered and does not abrogate the biological activity and properties of the compound. The pharmaceutical salts include acid addition salts formed by acids that form non-toxic acid addition salts containing a pharmaceutically acceptable anion, for example, inorganic acids such as hydrochloric acid, sulfuric acid, nitric acid, phosphoric acid, hydrobromic acid, hydroiodic acid, and the like, organocarbonic acid such as tartaric acid, formic acid, citric acid, acetic acid, trichloroacetic acid, trifluoroacetic acid, gluconic acid, benzoic acid, lactic acid, fumaric acid, maleic acid, salylic acid and the like, and sulfonic acid such as methanesulfonic acid, ethanesulfonic acid, benzenesulfonic acid, p-toluenesulfonic acid and the like. For example, pharmaceutically acceptable carboxylic acid salts include a metal salt or alkaline earth metal salt formed by lithium, sodium, potassium, calcium, magnesium and the like, salts of amino acids such as lysine, arginine, guanidine and the like, and organic salts such as dicyclohexylamine, N-methyl-D-glucamine, tris(hydroxymethyl) methylamine, diethanolamine, choline, triethylamine and the like.

As used herein, the term "disease with bone mass loss" refers to a disease or condition in which the reduction of bone mass, which leads to a symptom, such as a decrease in bone density and deterioration in bone tissue, occurs.

Examples of a disease with bone mass loss to which the composition of the present invention can be applied include osteopenia or osteoporosis. Specifically, the disease with bone mass loss may be any one of primary osteoporosis such as osteoporosis induced by menopause in women, senile osteoporosis, and osteoporosis induced by ovariectomy; secondary osteoporosis such as glucocorticoid-induced osteoporosis, hyperthyroid osteoporosis, fixation-induced osteoporosis, heparin-induced osteoporosis, immunosuppression-induced osteoporosis, osteoporosis due to renal insufficiency, inflammatory osteoporosis, osteoporosis accompanied by Cushing syndrome, and rheumatic osteoporosis; and bond disease such as hypercalcemia, Paget disease, bone defect, osteonecrosis, but is not limited thereto.

Preferably, the composition of the present invention can be used for the prevention or treatment of osteoporosis or osteopenia. Specifically, as used herein, the term "osteoporosis" means a condition in which bone mass decreases and quality of bone changes, leading to the increased risk of bone fracture. The term "osteopenia" refers to an initial condition of osteoporosis. Generally, patients with a bone density (T-score) of -1.0 to -2.5 are classified as having osteopenia, and patients with a T-value of -2.5 or lower are classified as having osteoporosis.

As used herein, the term "preventing" refers to all actions that inhibit a disease with bone mass loss or delay the development of the disease by administering the composition of the present invention.

As used herein, the term "treating" refers to all actions that alleviate or beneficially change the condition of a disease with bone mass loss by administering the composition of the present invention.

In an example of the present invention, it was found that the composition of the present invention effectively inhibited osteopenia caused by aging or ovariectomy (OVX). In addition, it was found that the administration of DDS significantly restored the reduction of bone density and bone mass. Also, high-resolution micro-CT images indicated that DDS of the present invention makes cancellous bone dense, thereby effectively controlling the reduction of bone density and bone mass.

For administration, the pharmaceutical composition of the present invention may comprise, in addition to the DDS or a pharmaceutically acceptable salt thereof, a pharmaceutically acceptable carrier, excipient or diluent. Examples of the carrier, excipient or diluent that may be used in the present invention include lactose, dextrose, sucrose, sorbitol, mannitol, xylitol, erythritol, maltitol, starch, acacia senegal gum, alginate, gelatin, calcium phosphate, calcium silicate, cellulose, methyl cellulose, microcrystalline cellulose, polyvinyl pyrrolidone, water, methyl hydroxybenzoate, propyl hydroxylbenzoate, talc, magnesium stearate, mineral oil, etc.

The pharmaceutical composition of the present invention may be formulated as oral dosage forms such as powders, granules, tablets, capsules, suspensions, emulsions, syrups or aerosol formulations, external dosage forms, suppositories, or sterile injectable solutions. Specifically, the composition of the present invention may be formulated with commonly used diluents or excipients, such as fillers, extenders, binders, wetting agents, disintegrants, surfactants, etc. Solid formulations for oral administration include, but are not limited to, tablets, pills, powders, granules, capsules and the like. Such solid formulations may comprise at least one excipient, for example, starch, calcium carbonate, sucrose, lactose or gelatin. In addition to simple excipients, lubricants such as magnesium stearate or talc may also be used. Liquid formulations for oral administration may contain various excipients, for example, wetting agents, flavoring agents, aromatics and preservatives in addition to water and liquid paraffin. Formulations for parenteral administration include sterilized aqueous solutions, non-aqueous solutions, suspensions, emulsions, freeze-dried preparations, and suppositories. As non-aqueous solvents or suspending agents, propylene glycol, polyethylene glycol, plant oils such as olive oil, injectable esters such as ethyl oleate, and the like can be used. As the base of the suppositories, witepsol, Macrogol, Tween 61, cacao butter, laurin fat, glycerogelatin and the like can be used.

The composition of the present invention may be administered orally or parenterally (e.g., intravenously, subcutaneously, intraperitoneally or topically) according to the intended use. The dose of the composition of the present invention may vary depending on the patient's condition and bodyweight, the severity of the disease, the form of drug, and the route and time of administration and can be suitably selected by those skilled in the art.

The present invention also provides a health functional food composition for preventing or ameliorating a disease with bone mass loss, comprising one or more of diaminodiphenylsulfone or a pharmaceutically acceptable salt thereof. The composition of the present invention may be administered alone or in combination with other therapeutic agents before or after the development of a disease with bone mass loss in order to prevent or ameliorate the disease with bone mass loss.

Preferably, the DDS may be autoclaved after mixing with water in order to further improve the water-insoluble property thereof before it is used. The autoclaving may be performed according to a general method known in the art. Preferably, the autoclaving may be performed at a temperature of 121 °C and a pressure of 151 psi for 15-20 minutes.

As used herein, the term "ameliorating" refers to all actions that at least reduce a parameter related to the condition to be treated, for example, the degree of symptom.

When the health functional food composition of the present invention is used as a food additive, the composition can be added alone or in combination with other foods or food ingredients, and may be used appropriately according to conventional methods. Generally, for the preparation of a food or a beverage, the composition of the present invention is added in an amount of 15 wt% or less, and preferably 10 wt% or less, based on the total weight of the food or beverage. However, for prolonged intake intended for the purpose of health and hygiene or for health control, the amount of the composition may be smaller than the lower limit of the above-specified range.

There is no particular limit to the kind of food. Examples of foods to which the composition of the present invention can be added include meats, sausages, bread, chocolate, candies, snack, confectionery, pizza, ramen, other noodles, gum, dairy products including ice cream, various soups, beverages, teas, drinks, alcoholic beverages and multivitamin preparations. The foods include all health foods in a conventional sense.

The health beverage composition of the present invention may additionally contain various sweetening agents or natural carbohydrates as in conventional beverages. The natural carbohydrates include monosaccharides, such as glucose and fructose, disaccharides, such as maltose and sucrose, natural sweeteners, such as dextrin and cyclodextrin, and synthetic sweeteners, such as saccharin and aspartame, may be used. The amount of the natural carbohydrates in the beverage composition can be suitably selected by a person skilled in the art.

In addition, the composition of the present invention may contain various nutrients, vitamins, electrolytes, flavoring agents, colorants, pectic acid or its salt, alginic acid or its salt, organic acids, protective colloidal thickeners, pH adjusters, stabilizers, preservatives, glycerin, alcohol, carbonating agents used in carbonated drinks, etc. Additionally, the composition of the present invention may contain fruit flesh for the preparation of natural fruit juices, fruit juice beverages and vegetable juices. These components may be used alone or in combination. The contents of these additives in the composition can be suitably selected by a person skilled in the art.

The present invention also provides a method for preventing or treating a disease with bone mass loss, comprising administering a pharmaceutically effective amount of the inventive composition, which comprises diaminodiphenylsulfone or a pharmaceutically acceptable salt thereof, to a subject having a disease with bone mass loss or bone density loss or being at risk of developing the same.

Preferably, the above method can be achieved by increasing bone density or bone mass.

As used herein, the term "subject" refers to all animals, including humans, who already have a disease with bone mass loss or bone density loss or are at risk of developing the same. The low-bond-density and disease with bone mass loss can be effectively prevented and treated by administering the composition to the subject.

The pharmaceutical composition of the present invention may be administered in a pharmaceutically effective amount. As used herein, the term "pharmaceutically effective amount" refers to an amount sufficient to treat diseases, at a reasonable benefit/risk ratio applicable to any medical treatment. The effective dosage level of the composition may be determined depending on the subject's type, the disease severity, the subject's age and sex, the type of the disease with bone mass loss, the activity of the drug, sensitivity to the drug, the time of administration, the route of administration, excretion rate, the duration of treatment, factors including drugs used in combination with the composition, and other factors known in the medical field. The pharmaceutical composition of the present invention may be administered alone or in combination with other therapeutic agents, and may be administered sequentially or simultaneously with conventional therapeutic agents. The composition can be administered in a single or multiple dosage form. It is important to administer the composition in the minimum amount that can exhibit the maximum effect without causing side effects, in view of all the above-described factors, and this amount can be easily determined by a person skilled in the art.

### [Mode for Invention]

Hereinafter, the present invention will be described in further detail with reference to examples. It is to be understood, however, that these examples are provided for illustrative purposes only and are not intended to limit the scope of the present invention.

### Example 1: Preparation of experimental animals with osteoporosis induced by ovariectomy

10-week-old BALB/c mice were subjected to ovariectomy (OVX) to make experimental animal models with osteopenia induced by OVX. In order to analyze bone decrease at 12 weeks after OVX at which osteopenia appears, DDS was administered orally at a dose of 2 mg/kg over a period ranging from one week before ovariectomy to 12 weeks after ovariectomy. Mice were divided into the following three groups, each consisting of 8 mice: a negative control group (untreated group), a positive control group (a group mice subjected to ovariectomy), and an experimental animal group subjected to ovariectomy and administered with DDS at the same time. All the animal studies were approved by the Animal Research Ethical Committee of Seoul National University.

### Example 2: Analysis of effects of DDS on control of BMD and BMC by dual energy X-ray absorptiometry (DEXA)

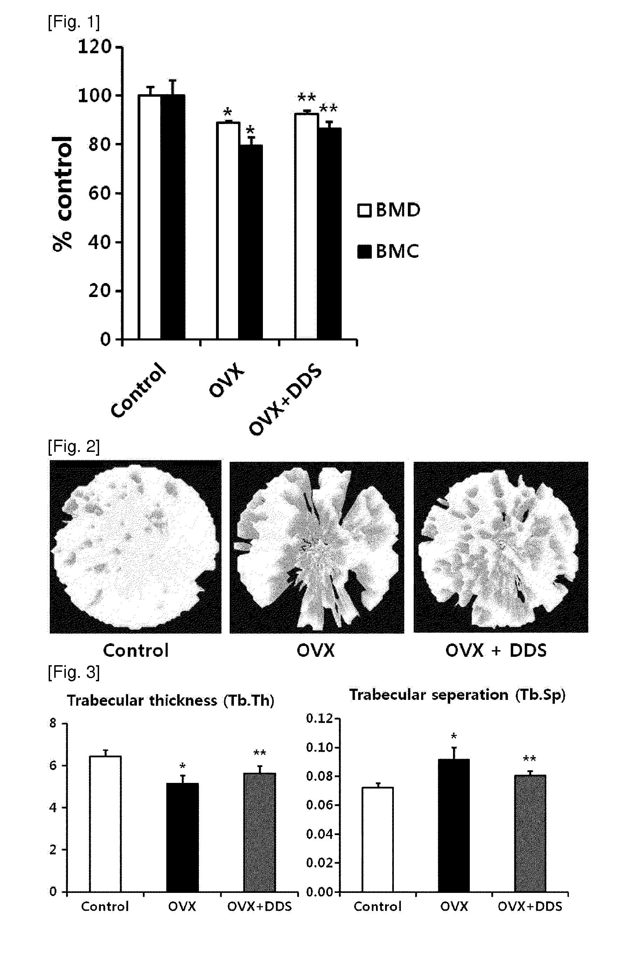

In order to examine whether DDS has the effect of controlling osteoporosis, the bone mineral density and bone mass of the whole body of the ovariectomized mice were measured by dual energy X-ray absorptiometry, and the results of the measurement are shown in FIG. 1.

As can be seen in FIG. 1, at 12 weeks after ovariectomy, the bone mineral density and bone mass of the positive control group (OVX) were significantly reduced to 88.9% and 79.3%, respectively, compared to that of the negative control group (100%). However, the OVX experimental group administered with DDS showed an increase in bone mineral density of 4.09% and an increase in bone mass of 9.13% compared to the positive control group (*P value < 0.05 for the negative control group; ** P value < 0.05 for the positive control group). This suggests that DDS of the present invention effectively induces bone regeneration to restore the reduction of bone mineral density and bone mass.

### Example 3: High-resolution micro-CT imaging and measurement of travecular thickness (Tb. Th) and trabeular separation (Tb. Sp) of femoral region of mice

The trabecular bone of femoral region adjacent to the mouse shin was imaged by radiography using high-resolution micro-CT. The right limb of the mice was excised and stored in 10% formalin for one day. Then, the bone tissue was stored in 70% alcohol at 4 °C until use in analysis. The bone tissue was imaged by computer tomography using Skyscan 1076 Desktop X-ray Microtomograph (Skyscan, Belgium) at a tube voltage of 100 kV and an exposure current of 200 µA. For the acquired images, bone mineral density (BMD) and bone volume (BV) were measured using Skyscan CTan ver. 1.5.0 and Shortcut to ANT image analysis program (Skyscan, Belgium), and the acquired images were reconstituted into three-dimensional images. The results of the reconstitution are shown in FIG. 2. In addition, trabecular thickness (Tb. Th) and trabecular separation (Tb. Sp), which are the indices of morphological microstructures on the micro CT images, were measured, and the results of the measurement are shown in FIG. 3.

As can be seen from the three-dimensional structure in FIG. 2, the microstructure of the bone section in OVX subjected group became significantly looser than that in the negative control group, suggesting that OVX induces osteopenia or osteoporosis. Meanwhile, the bone section of the OVX experimental group administered with DDS was as dense as that of the negative control group, suggesting that DDS effectively inhibits bone loss.

As can be seen in FIG. 3, the results of measurement of trabecular thickness (Tb. Th) and trabecular separation (Tb. Sp), which are the indices of morphological microstructures on the micro CT images, were compared, and as a result, it could be seen that OVX increased Tb. Th and decreased Tb. Sp. Meanwhile, it was shown that the OVX experimental group administered with DDS was controlled Tb. Th to be increased and Tb. Sp to be decreased to levels similar to those of the negative control group (*P value < 0.05 for negative control; ** P value < 0.05 for positive control (OVX treated group)).

### Example 4: Preparation of experimental animals with osteoporosis induced by aging

In order to examine the effect of DDS in animal models with osteopenia induced by aging, eight 5-month-old female C57BL/6J mice and eight 27-month-old female mice were purchased from the Korea Research Institute of Bioscience and Biotechnology. The mice were acclimated for 1 week, housed at a temperature of 21±1°C and a humidity of 60%, and allowed access to food *ad libitum.* DDS was administered to the mice at a dose of 2 mg/kg over 12 weeks in order to examine the effect of DDS against osteoporosis.

### Example 5: Examination of effects of DDS on control of BMD and BMC by dual energy X-ray absorptiometry (DEXA)

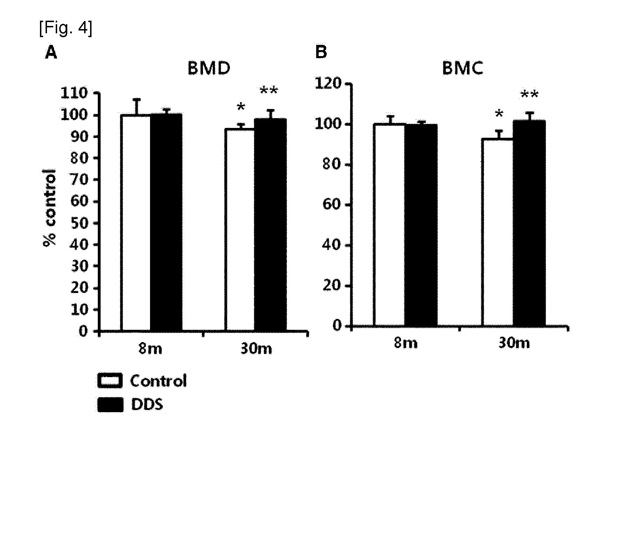

In order to examine whether DDS has the effect of controlling the osteoporosis or osteopenia caused by aging, changes in the bone mineral density and bone mass of the whole body of mice were measured by dual energy X-ray absorptiometry, and the results of the measurement are shown in FIG. 4.

As a result, as can be seen in FIG. 4, in the case of the control group not treated with DDS, the bone mineral density and bone mass of the 30-month-old mice were reduced to 93.32% and 92.71%, respectively, compared to those of the 8-month-old mice (100%). On the other hand, in the groups administered with DDS, no significant change in the bone mineral density or bone mass of the 8-month-old mice was observed, but the bone mineral density and bone mass of the 30-month-old mice increased by 5.11% and 9.6%, respectively. This suggests that DDS has the effect of inhibiting osteopenia or osteoporosis by effectively restoring bone mineral density and bone mass.

## Claims

1. A pharmaceutical composition for preventing or treating a disease with bone mass loss, comprising diaminodiphenylsulfone or a pharmaceutically acceptable salt thereof.

2. The pharmaceutical composition of claim 1, wherein the disease with bone mass loss is osteoporosis or osteopenia.

3. The pharmaceutical composition of claim 1, wherein the disease with bone mass loss is osteoporosis induced by menopause in women, senile osteoporosis, or osteoporosis induced by ovariectomy.

4. The pharmaceutical composition of claim 1, wherein the diaminodiphenylsulfone is autoclaved at a pressure of 151 psi.

5. A health functional food composition for preventing or ameliorating a disease with bone mass loss, comprising diaminodiphenylsulfone or a pharmaceutically acceptable salt thereof.

6. The health functional food composition of claim 5, wherein the disease with bone mass loss is osteoporosis or osteopenia.

7. The health functional food composition of claim 5, wherein the disease with bone mass loss is osteoporosis induced by menopause in women, senile osteoporosis, or osteoporosis induced by ovariectomy.

8. The health functional food composition of claim 5, wherein the diaminodiphenylsulfone is autoclaved at a pressure of 151 psi.

9. A method for preventing or treating a disease with bone mass loss, the method comprising administering the pharmaceutical composition of any one of claims 1 to 4 to an animal except for a human.
